# EUROPEAN PATENT APPLICATION

(11) **EP 0 898 937 A1**
(43) Date of publication of application: **03.03.1999**
(21) Application number: 98306962.6
(22) Date of filing: 28.08.1998
(51) Int. Cl.: A61B 6/08

(54) **Method and device for positioning a radiology instrument**

(30) Priority: 01.09.1997 FR 9710861
(71) Applicant: GE MEDICAL SYSTEMS SA, 78533 Buc Cedex (FR)
(72) Inventor: Romeas, Rene, 91120 Palaiseau (FR); Maurincomme, Eric, 78530 Buc (FR); Delgado, Claude, 78370 Plaisir (FR)
(74) Representative: Goode, Ian Roy

(57) **Abstract**

Positioning device for a radiology instrument of the type comprising a means for emitting an X-ray beam, a means for receiving the X-ray beam after it has passed through a radiographed body in order to obtain an image of the said body, a support (6) for the emission means and the reception means, this support being capable of moving in rotation about at least two secant axes, and a table (14) which supports the said body and can move in translation along three orthogonal axes. The device comprises a light source (19), at least one semi-silvered mirror (21) and a target (22), the light source (19) and the target (22) being secured to the support (6) of the emission means and the reception means or the table (14), the semi-silvered mirror (21) being respectively secured to the table (14) or to the support (16) of the emission means and the reception means, so that the light beam emitted by the source (19) is reflected on the mirror (21) and reaches the target (22), the support (6) of the emission means and the reception means being positioned in rotation when the reflected beam reaches a given point of the target (22), and the table (14) being positioned in translation when the incident beam reaches a given point of the mirror (21).

## Description

The present invention relates to the field of the precise and reproducible positioning of two components with respect to one another, in particular two components of a radiology instrument.

A radiology instrument, for example for neurological or vascular use, is generally composed:
of an X-ray tube and a collimator for forming and delimiting the X-ray beam,
of an image receiver, generally a radiological image intensifier plus a video camera,
of a positioner which carries the assembly consisting of the X-ray tube and the collimator, as well as the radiological image intensifier and the video camera, and can move in space about at least two orthogonal axes, possibly about three axes, and
of a table provided with a plate which is intended to support the patient in a recumbent position.

In the case when the positioner is provided with three axes of rotation, the orientation in space of two axes depends on the angle of rotation about a vertical axis. These angles of rotation are orthogonal to one another and intersect at a point which is referred to as the isocentre of the positioner. This configuration makes it possible to orientate the axis of the X-ray beam in almost all directions in space. The plate of the table can be moved along three orthogonal axes so as to position the patient correctly with respect to the isocentre of the positioner.

The X-ray tube is powered by a high-voltage generator, and the images output by the video camera are processed, displayed and stored in an image processing system.

A radiological instrument of this type may, in particular, be used for performing interventions used in a device which is referred to as a stereotaxy frame. This device makes it possible to immobilize the patient's head using four metal points inserted into the cranium in small holes drilled into the bone by the surgeon. The stereotaxy frame is fixed solidly to the plate of the table and may be provided with radiopaque locating elements which makes it possible for the anatomical structures to be located with precision in space. A device of this type allows the patient's head to be repositioned precisely with respect to the frame. This positioning is essential if the patient is to undergo several successive examinations, which is the case in certain treatments and operations.

It is therefore necessary to reposition the radiological system with precision with respect to the stereotaxy frame, in particular for taking orthogonal front and side radiology exposures, for example to check for correct positioning of the tools or instruments during an operation.

Radiological tables and positioners are generally equipped with systems for displaying positions, linear positions for the table and angular positions for the positioner. These values of the positions of the table and angles of the positioner are given either with respect to a reference which is determined on installation, or with respect to a reference which is determined by the user according to his requirements. The positions may be measured using potentiometric sensors or the like which are mounted on the equipment items.

Repositioning of the radiological system in a given configuration may take place in two ways:
- either in manual mode, with the operator himself controlling the movement of the various elements by monitoring the indications of the position display device until the desired positions are obtained,
- or in automatic mode, for the Most advanced instruments, by storing the positions of the various axes and recovering them automatically at the operator's request.

Irrespective of the positioning mode which is used, the precision obtained is about plus or minus 1 mm for the positions of the table and plus or minus one degree for the angles of the positioner, under optimum circumstances. This is due to the set of errors introduced in the measuring system: resolution of the sensors, transmission play, etc. Furthermore, these values do not take into account flexions of the table plate and other possible deformations under load of the table and the positioner.

A more precise check of the positioning can be made by using radiopaque alignment markers, but this makes it necessary for the operator to carry out the operations of repositioning the radiological system under fluoroscopy, that is to say under the emission of X-rays, which entails additional irradiation of the patient and the operator.

It is therefore desirable to overcome the drawbacks of existing devices by proposing a device and a method for positioning which are precise and which overcome the various errors of the measuring system.

A further need is to reduce the exposure of the patient and the operator to X-rays.

The positioning device is intended for a radiology instrument of the type comprising a means for emitting an X-ray beam, a means for receiving the X-ray beam after it has passed through a radiographed body in order to obtain an image of the said body, a support for the emission means and the reception means, this support being capable of moving in rotation about at least two secant axes, and a table which supports the said body and can move in translation. The positioning device comprises a light source, at least one mirror and a target, the light source and the target being secured to the support of the emission means and the reception means or the table, the mirror being respectively secured to the table or to the support of the emission means and the reception means, so that the light beam emitted by the source is reflected on the mirror and reaches the target, the support of the emission means and the reception means being positioned in rotation when the reflected beam reaches a given point of the target, and the table being positioned in translation when the incident beam reaches a given point of the mirror. The use of the positioning device thus involves no additional irradiation. Satisfactory maintenance of the positioning may be checked at any time, including during an operation.

Advantageously, in one embodiment the device comprises two perpendicular mirrors. Positioning can thus be carried out in two perpendicular planes.

In one embodiment of the invention, the light source is arranged at the centre of the target.

In one embodiment of the invention, the mirror is semi-silvered and provided with a face for receiving the light beam, and with a face opposite the reception face.

The opposite face is provided with a reticle. It is thus possible to check centring of the incident beam on the mirror on the side of the opposite face.

Advantageously, in one embodiment the light source and the target or the mirror are mounted on a stereotaxy frame secured to the table.

In one embodiment of the invention, the stereotaxy frame comprises at least one radiopaque locating element. The stereotaxy frame may also comprise means for fixing a part of a patient's body.

In one embodiment of the invention, the mirror is mounted on an edge of the stereotaxy frame, so that the distance separating the centre of the said mirror and the X-ray beam is equal to the distance separating the light source and the X-ray beam. This avoids parallax errors.

The light source and the target are preferably secured to the support of the emission means and the reception means and are adjacent to the means for emitting the X-ray beam, and the mirror is secured to the table. This table may be capable of moving along three orthogonal axes.

Advantageously, in one embodiment the light source is a diode laser.

In one embodiment of the invention, the incident light beam is parallel to the X-ray beam.

The invention also relates to a radiology instrument comprising a positioning device as described above.

The positioning method is intended for a radiology instrument of the type comprising a means for emitting an X-ray beam, a means for receiving the X-ray beam after it has passed through a radiographed body in order to obtain an image of the said body, a support for the emission means and the reception means, this support being capable of moving in rotation about at least two secant axes, and a table which supports the said body and can move in translation. The method comprises the following steps:
- emitting a light beam from an element of the support, the light beam being reflected by a first element of the table towards the support,
- orienting the support so that the reflected light beam reaches a given point of the support,
- centering the incident light beam on a given point of the first reflection element of the table,
- rotating the support by an angle of π/2 radians with respect to a longitudinal axis of the table,
- emitting a light beam from the element of the support, the light beam being reflected by a second element of the table towards the support,
- orienting the support so that the reflected light beam reaches the given point of the support,
- centering the incident light beam on a given point of the second reflection element of the table.

A relative position of the table and the means for emitting an X-ray beam can thus be reproduced precisely, independently of the resolution of the sensors, transmission plays and flexion and the possible deformations of the plate of the table and the elements supporting the means for emitting the X-ray beam.

The invention will be understood more clearly on studying the detailed description of an embodiment, taken by way of an entirely non-limiting example, and illustrated by the appended drawings, in which:
Figure 1 is a perspective view of a radiology instrument;
Figure 2 is a detailed perspective view of a radiology instrument having an embodiment of the positioning device; and
Figure 3 is a similar view to Figure 2, showing the radiology instrument in a position in which it is offset by an angle of π/2 with respect to Figure 2.

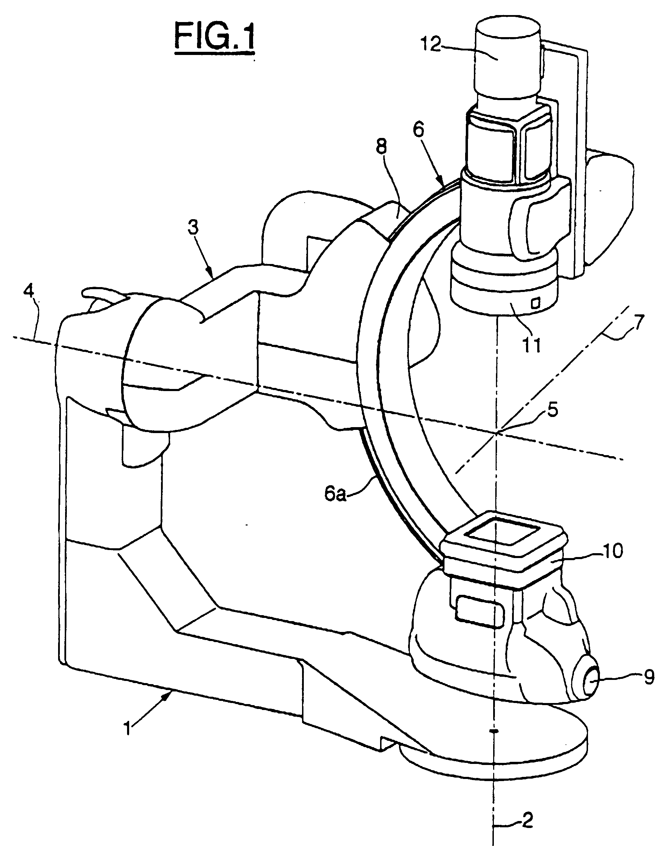

Figure 1 shows a radiology instrument having a base 1 which can move in rotation, about a vertical axis 2, with respect to a framework (not shown) secured to the floor. An arm 3 is rotationally articulated with respect to the base 1 about a horizontal axis 4. The vertical 2 and horizontal 4 axes are secant orthogonally at the point 5, referred to as the isocentre of the radiology instrument. A support element 6 is rotationally articulated onto the arm 3 about an axis 7 which is orthogonal to the axis 4 and passes through the isocentre 5. The support element 6 is provided with a rail 6a, in the shape of an arc of a circle, which interacts with a jaw system 8 of the arm 3 in order to allow the support element 6 to rotate with respect to the said arm 3. The rail 6a may be provided with a rack (not shown) capable of interacting with toothed wheels (not shown) of the arm 3.

The lower end of the support element 6 is secured to an X-ray tube 9 and a collimator 10 which is intended to form and delimit the X-ray beam. The X-ray tube 9 is connected to electrical power supply means and to control means (not shown). The upper end of the support element 6 is secured to a radiological image intensifier 11 and a video camera 12. The video camera 12 is connected to an electrical supply and to image processing means (not shown).

In order to ensure suitable imaging, that part of the patient's body which is to be radiographed should be placed in the path of the X-ray beam between the collimator 10 and the radiological image intensifier 11, immediately next to the isocentre 5.

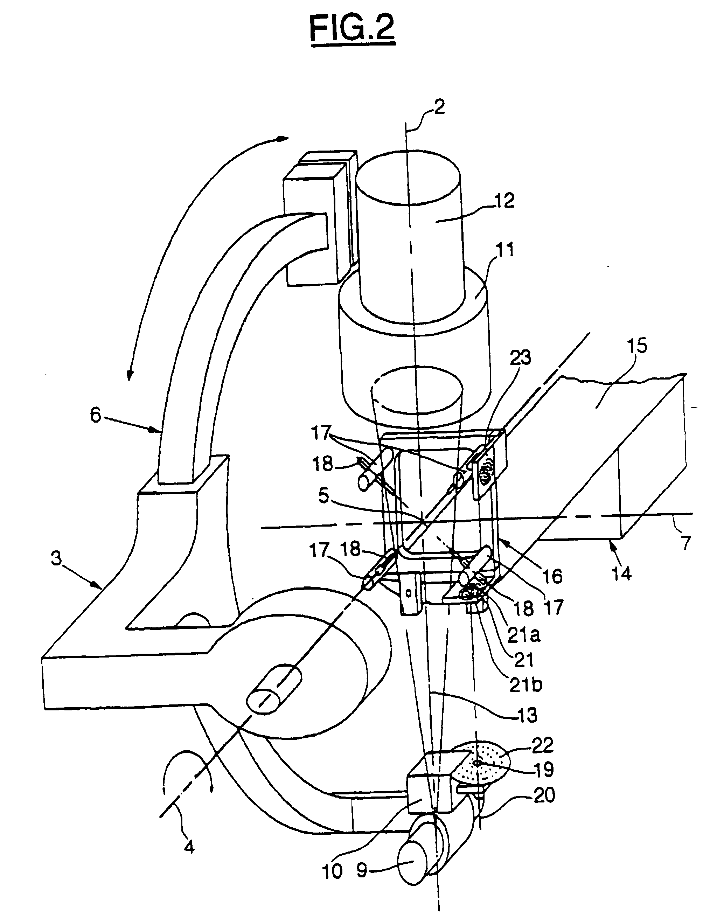

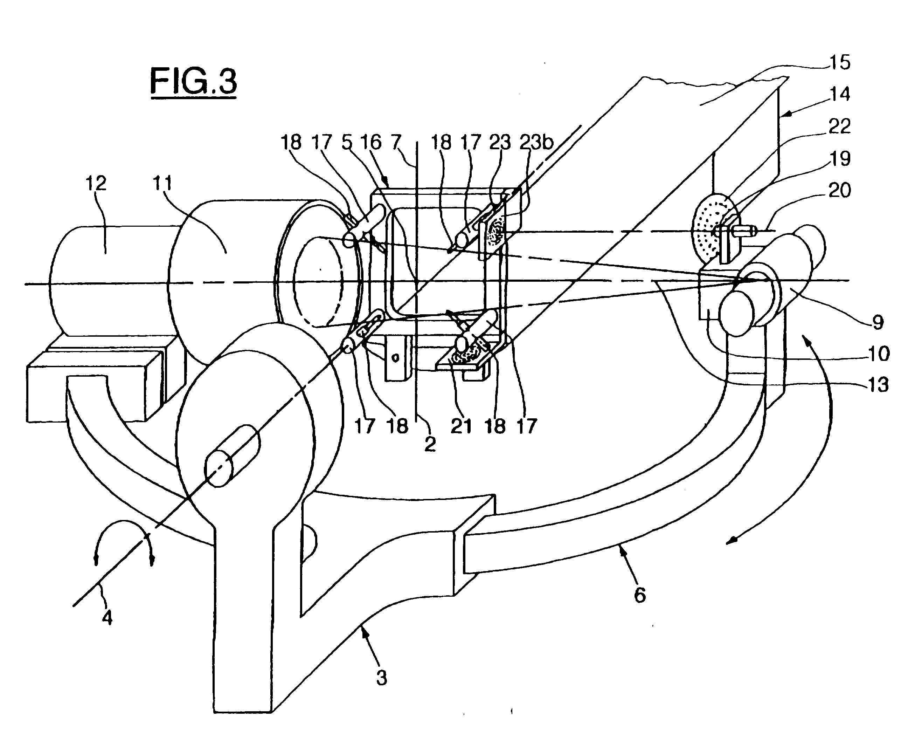

In Figures 2 and 3, the X-ray beam is emitted from the collimator 10 along a reference axis 13. A table 14, comprising a plate 15, is provided in order to support the patient (not shown) . The plate 15 can move in translation along three mutually orthogonal axes. The desired part of the patient's body can thus be brought close to the isocentre 5.

For treating or operating on a patient's head, the greatest possible precision is required. In the case of treating epilepsy, the afflicted region of the brain is sought using probes inserted into the patient's head. When these probes are inserted, it is essential to avoid the blood vessels which supply the brain. This makes it necessary, on the one hand, to locate them properly and, on the other hand, to find the location where the probe is to be placed, the intervention generally being performed after radiology and MRI sessions which are intended for in-depth study of the brain.

To this end, use is made of a stereotaxy frame 16 which is fixed to the free end of the plate 15 and is arranged vertically. The stereotaxy frame 16, which is rectangular, comprises four columns 17, each arranged at one corner of the frame 16 and each provided with screws 18 which are arranged in a vertical plane and are oriented towards the axis 4. These screws 18 allow the patient's head to be immobilized, the ends of these screws being inserted into the cranium in small holes drilled into the bone by the surgeon. This ensures precise positioning of the patient's head with respect to the stereotaxy frame 16. In order for the positioning of the screws 18 to be found precisely, they are provided with graduations.

A light source 19 is mounted on the side of the collimator 10 and secured to it, this source being, for example, a diode laser emitting a light beam which is parallel to the axis 13 of the X-ray beam and is directed towards the stereotaxy frame 16. The light beam originating from the source 19 propagates along an axis 20 in the direction of a semi-silvered mirror 21 secured to the stereotaxy frame 16. The mirror 21 is mounted in such a way as to be perpendicular to the frame 16 and substantially horizontal, in extension of the plate 15. The beam reflected by the mirror 21 is directed towards the light source 19 and strikes a circular target 22 arranged around the light source 19, which forms its centre.

The mirror 21 is provided with a reticle formed by concentric circles drawn on that face 21a of the mirror 21 which is opposite the face 21b for receiving the light beam output by the source 19. An operator can thus perceive the mark due to the light beam on the opposite face 21a of the mirror 21 and use the reticle to assess the centring of the light beam. In order to avoid parallax errors, the centre of the mirror 21 is mounted on the stereotaxy frame 16 in such a way that the distance separating it from the vertical axis 2 is equal to the distance separating the light source 19 from the axis 13 along which the X-ray beam is emitted, the axes 2 and 13 being merged in the position in Figure 2.

After the patient's head has been fixed on the stereotaxy frame 16, and before any X-rays have been emitted, the light source 19 is used to send a light beam along the axis 20. The mark due to the reflected light beam on the target 22 is located, then the orientation of the arm 3 and the support element 6 is modified so that the reflected light beam reaches a given point of the target 22, for example the emission point of the light source 19.

The mark due to the incident light beam on the reticle of the mirror 21 is located. The plate 15 is moved in translation so that the incident light beam reaches the centre of the reticle. The stereotaxy frame will in this way have been positioned precisely with respect to the X-ray emission means in a substantially vertical plane. In fact, this plane corresponds to a plane passing through an axis orthogonal to the mirror 21, which may deviate slightly from the horizontal because of the flexing of the plate 15 and the play which may occur in the table 14.

The arm 3 is then turned by an angle of π/2 radians with respect to the axis 4. The radiology instrument thus assumes the position illustrated in Figure 3, the axes 2 and 7 being merged. The same positioning operation is repeated with respect to a second mirror 23, identical to the first mirror 21 but arranged on a vertical edge of the stereotaxy frame 16 so as to have a reception face 23b which is substantially vertical and perpendicular to the axis 20 of the incident light beam in the position in Figure 3. After having carried out the angular centering and the linear centering in the position illustrated in Figure 3, the X-ray emission means is positioned between two orthogonal planes with respect to the stereotaxy frame. This provides a positioning precision to within 0.5 nm for the three axes of the table plate, and to within 0.1 of a degree for the axes of rotation of the radiology instrument.

Of course, other positions can be obtained by placing mirrors in a different way on the stereotaxy frame 16, or by adding additional mirrors. The mirror or mirrors could also be fixed on an element other than the stereotaxy frame 16, for example the plate 15.

In order to complete this positioning, it may be supplemented by the technique of locating using radiopaque elements, for example by placing a lead ball, secured to the collimator, in the X-ray beam and by aligning it with a hole in a metal sheet secured to the plate.

It is also possible to leave the light source 19 on throughout the operation, which makes it possible to monitor a possible change in position and consequently to return to the desired position. The positioning may also be carried out using an automatic system, for example with the aid of CCD cameras which locate the mark of the light beam on the mirror 21 and on the target 22 and, by means of a control and regulating device, acting on the motors of the base 1, the arm 3, the support element 6 and the table 14. Automatic repositioning may thus be carried out, which makes it possible to save time and reduce the operator's work load.

By virtue of the invention, the X-ray emitter is positioned with respect to one of the patient's organs while overcoming the inaccuracies due to the flexing of the mechanical components and the resolution of the sensors of various components, which affords a considerable improvement in precision.

## Claims

1. A positioning device for an instrument of the type comprising a means for emitting a beam, a means for receiving the beam after it has passed through a body in order to obtain an image of the body, a support (6) for the emission means and the reception means, this support being capable of moving in rotation about at least two axes, and a table (14) which supports the body and can move in translation, comprising a light source (19), at least one mirror (21) and a target (22), the light source and the target being secured to the support of the emission means and the reception means or the table, the mirror being respectively secured to the table or to the support of the emission means and the reception means, so that the light beam emitted by the source is reflected on the mirror and reaches the target, the support of the emission means and the reception means being positioned in rotation when the reflected beam reaches a given point of the target, and the table being positioned in translation when the incident beam reaches a given point of the mirror.

2. Device according to claim 1 comprising two perpendicular mirrors.

3. Device according to either one of the preceding claims wherein the light source is arranged at the centre of the target.

4. Device according to any one of the preceding claims wherein the mirror (21) is semi-silvered and provided with a face (21b) for receiving the light beam, and with a face (21a) opposite the reception face, the opposite face being provided with a reticle.

5. Device according to any one of the preceding claims wherein the light source and the target or the mirror are mounted on a stereotaxy frame (16) secured to the table.

6. Device according to Claim 5 wherein the stereotaxy frame comprises at least one radiopaque locating element.

7. Device according to either one of Claims 5 and 6 wherein the mirror is mounted on an edge of the stereotaxy frame, so that the distance separating the centre of the mirror and the beam is equal to the distance separating the light source and the beam.

8. Device according to any one of the preceding claims wherein the light source and the target are secured to the support- of the emission means and the reception means and are adjacent to the means for emitting the beam, and the mirror is secured to the table.

9. Device according to any one of the preceding claims wherein the light source is a diode laser.

10. Device according to any one of the preceding claims wherein the incident light beam is parallel to the beam.

11. Radiology instrument comprising a positioning device according to any one of the preceding claims.

12. A method for positioning an instrument of the type comprising a means for emitting a beam, a means for receiving the beam after it has passed through a body in order to obtain an image of the body, a support for the emission means and the reception means, this support being capable of moving in rotation about at least two axes, and a table which supports the body and can move in translation comprising the steps of:
- emitting a light beam from an element of the support, the light beam being reflected by a first element of the table towards the support,
- orienting the support so that the reflected light beam reaches a given point of the support,
- centering the incident light beam on a given point of the first reflection element of the table,
- rotating the support by an angle of π/2 radians with respect to a longitudinal axis of the table,
- emitting a light beam from the element of the support, the light beam being reflected by a second element of the table towards the support,
- orienting the support so that the reflected light beam reaches the given point of the support; and
- centering the incident light beam on a given point of the second reflection element of the table.
